# EUROPEAN PATENT SPECIFICATION

(11) **EP 2 836 200 B1**
(45) Date of publication and mention of the grant of the patent: **15.07.2020**
(21) Application number: 13775035.2
(22) Date of filing: 08.04.2013
(51) Int. Cl.: A61K 9/00, A61K 47/10, A61K 47/32, A61K 47/38, A61K 38/48, A61P 21/00

(54) **REVERSE THERMAL HYDROGEL PREPARATIONS FOR USE IN THE TREATMENT OF DISORDERS OF THE UROTHELIUM**
THERMOREVERSIBLE HYDROGELPRÄPARATE ZUR BEHANDLUNG VON ERKRANKUNGEN DES UROTHELS
PRÉPARATIONS D'HYDROGEL THERMORÉVERSIBLE POUR LEUR UTILISATION DANS LE TRAITEMENT DE TROUBLES DE L'UROTHÉLIUM

(30) Priority: 08.04.2012 US 201261621525 P
(43) Date of publication of application: 18.02.2015
(73) Proprietor: Urogen Pharma Ltd., 4365007 Ra'anana (IL)
(72) Inventor: HAKIM, Gil, 43596 Ra'anana (IL); KONORTY, Marina, 46446 Hertzlya (IL); JESHURUN, Michal, 4481400 Elkana (IL)
(74) Representative: Pohlman, Sandra M.
(86) International application number: PCT/IL2013/050314
(87) International publication number: WO 2013/153550

(56) References cited:
- EP-B1- 1 663 217
- WO-A1-2006/065234
- WO-A1-2013/011504
- WO-A2-2011/018800
- WO-A2-2011/089604
- WO-A2-2011/089604
- US-A- 3 069 411
- US-A1- 2004 009 212
- US-A1- 2006 257 488
- US-A1- 2007 269 379
- US-A1- 2007 275 110
- US-A1- 2008 317 765
- US-A1- 2009 214 685
- US-A1- 2009 304 747
- US-A1- 2010 036 000
- US-B1- 6 365 164
- US-B2- 7 780 958
- ESCOBAR-CHAVEZ J : ET AL: "Applications of thermo-reversible pluronic F-127 Gels in Pharmaceutical Formulations", JOURNAL OF PHARMACY & PHARMACEUTICAL SCIENCES, CANADIAN SOCIETY FOR PHARMACEUTICAL SCIENCES, CA, vol. 9, no. 3, January 2006 (2006-01), pages 339-358, XP002415851,

## Description

### FIELD OF THE INVENTION

This invention relates in general to preparations for use in the treatment of bladder disorders that result from dysfunction of the urothelium. It relates in particular to preparations that incorporate an active ingredient such as botulinum toxin in a reverse thermal hydrogel.

### BACKGROUND OF THE INVENTION

Disorders of the urothelium that result in Urinary Incontinence (UI) affect 200 million people worldwide. The United States National Institutes of Health has estimated that as many as 25 million adult Americans have experienced or will experience transient or chronic UI. The National Association of Continence estimates that 75-80% of these sufferers are women, 9-13 million of whom have bothersome to severe symptoms. It has been estimated that one in four women over the age of 18 experiences episodes of involuntary leakage of urine. One-third of men and women aged 30-70 have experienced loss of bladder control at some point in their adult lives and may be still living with the symptoms. Of men and women ages 30-70 who awaken during the night to use the bathroom, more than one-third get up twice or more per night to urinate, fitting the clinical diagnosis of nocturia. Of these adults, one in eight report they sometimes lose urine on the way to the bathroom. Overactive bladder (OAB) is especially common in older adults. It affects an estimated 1 in 11 adults in the United States. Currently available treatment options for OAB include bladder training; pelvic floor exercises; administration of drugs such as anti-cholinergics, capsaicin, and intravesical botulinum toxin injections; and, in severe cases, bladder augmentation surgery.

Another common disorder of the urothelium is Interstitial Cystitis, a chronic inflammation of the bladder that causes chronic pain and discomfort, which affects an estimated 4 million people in the U.S. alone.

The main shortcoming of the orally-administered drugs in current use is their high adverse event rate resulting in patients' intolerability. Alternative routes of administration with lower adverse event rates, such as transdermal and via suppository, have been developed. Bladder instillation of antimuscarines also has a lower adverse event rate than oral administration, but suffers from the disadvantage that it requires recurring catheterization due to the relatively short half-life of the antimuscarines, thus reducing compliance.

Another approach that has been developed recently is the use of intravesical administration of botulinum toxin. Despite the promising results of this therapeutic approach, numerous drawbacks remain. For example, the cystoscopic injection requires technical proficiency and a physician's authorization; administration of an anesthetic is required; botulinum toxin affects only the tissue at the location where it has been injected; and the treatment may lead to temporary urinary retention and consequently may require self-catheterization.

U.S. Pat. No. 8277822 discloses a composition and method for treatment of pathologies characterized by bladder spasms such as overactive bladder, interstitial cystitis, stress incontinence, urge incontinence, and neurogenic bladder in which the composition is administered by infusion into the bladder. According to the method disclosed therein, a liquid, semi-solid, or solid composition comprising botulinum toxin is spread on the outer surface of a balloon. The balloon is then introduced into the bladder via an endoscopic or cystoscopic apparatus or via a catheter and inflated until it contacts the inner wall of the bladder, thereby depositing the composition on the inner wall of the bladder.

U.S. Pat. Appl. Pub. No. 2009/0214685 (henceforth **'685**) discloses a sustained release poloxamer composition containing botulinum toxin. The botulinum toxin is incorporated into a thermoreversible thermoplastic poloxamer composition which is introduced via injection into the bladder as a liquid, and gels after administration to provide a sustained release delivery system. Even though the relevant physical properties of thermoreversible poloxamer compositions, in particular, the temperature-dependent viscosity, tend to be quite sensitive functions of the poloxamer composition, the compositions disclosed in **'685** provide only a limited range of viscosities for both the liquid and the gel state of the composition, probably due to the need for sufficiently low viscosity to enable withdrawal of the formulation into a syringe and injection into the patient. Furthermore, **'685** does not provide any teaching regarding the mucoadhesiveness of the compositions disclosed therein.

There thus remains a long-felt need for compositions for delivery of therapeutic agents such as botulinum toxin for treatment of bladder disorders in which the viscosity is sufficiently low at low temperature to permit administration by direct instillation into the bladder onto the urothelium rather than by intramuscular injection, but that have a mucoadhesiveness sufficiently high that the composition remains attached to the bladder wall long enough for sustained-release delivery of the therapeutic agent with an enhanced efficacy.

### SUMMARY OF THE INVENTION

The compositions disclosed herein, for use in treatments of bladder disorders and urothelium dysfunction are designed to meet this long-felt need. In particular, mucoadhesive, bioerodible, biocompatible thermoreversible hydrogel compositions comprising a therapeutically active agent are disclosed that after insertion into a body cavity, solidify and form a drug reservoir inside the cavity. The compositions and means for delivering them produce complete contact with and coverage of the bladder wall, thereby providing drug delivery to the entire bladder, in contrast to delivery induced by local injections. They additionally produce a high topical drug concentration in the cavity walls but low systemic exposure. By these means the invention herein disclosed provides increased bioavailability enhanced efficacy and reduced toxicity.

The invention is directed to a biocompatible mucoadhesive thermoreversible hydrogel composition incorporating botulinum toxin and comprising at least one reverse thermal gelation agent which is a poloxamer, and at least one component selected from the group consisting of mucoadhesive enhancers and thickening compounds, selected from the group consisting of polycarbophil, polyacrylic acids, crosslinked acrylic acid, polymethacrylates, divinyl glycol, polyethylene glycol (PEG), polyethylene oxide, vinylpyrrolidone/vinyl acetate copolymer, cellulose, microcrystalline cellulose, cellulose derivatives, gelatin, starch, starch derivatives, gums, dicalcium phosphate, lactose, sucrose, polyvinylpyrolidone (PVP), polyvinyl alcohols (PVA), partially hydrolysed polyvinyl acetate (PVAc), polysaccharides, waxes, fats, fatty acid derivatives, hyaluronic acid, chitosan, K-carrageenan, carbomers, Eudragit, and any combination thereof for use in the treatment of a bladder disorder, wherein the bladder disorder is characterized by bladder spasms, wherein the treatment comprises applying the composition to a bladder cavity, and wherein the application does not comprise any step of injecting botulinum toxin into a wall of said bladder.

Further embodiments of the invention are specified in the claims.

Aspects and embodiments disclosed herein which do not fall within the scope of the claims do not form part of the invention.

Disclosed herein are thermoreversible hydrogel compositions suitable for maintaining and/or enhancing the efficacy of a pharmaceutically active agent in a sustained-release treatment of a bladder disorder, by obtaining a biocompatible mucoadhesive thermoreversible hydrogel; and, incorporating said pharmaceutically active agent for treatment of a bladder disorder into said biocompatible mucoadhesive thermoreversible hydrogel.

Disclosed herein is a thermoreversible hydrogel composition for use in treating a bladder disorder, wherein the treatment comprises applying to a bladder cavity a biocompatible mucoadhesive thermoreversible hydrogel into which a pharmaceutically active agent for treating said bladder disorder has been incorporated, wherein said application does not comprise any step of injecting a pharmaceutically active agent into a wall of said bladder.

The pharmaceutically active ingredient for treatment of a bladder disorder incorporated into the biocompatible mucoadhesive thermoreversible hydrogel, is selected from the group consisting anticholinergic agents, antimuscarinic agents, and beta-3 agonists. The pharmaceutically active agent for treatment of a bladder disorder is selected from the group consisting of oxybutynin, tolterodine, fesoterodine fumarate, solifenacin, pentosan polysulfate, amitriptyline, heparin-binding epidermal growth factor (HB-EGF), all-trans-retinoic acid (ATRA), derivatives of antiproliferative factor (APF), cannabinoid-2 receptor blockers, memantine, N-methyl-D-aspartate (NMDA)-receptor blockers, Parthenolide, inhibitors of NF-κB, calcium glycerophosphate, Metamucil, and Chondroitin sulfate.

In the embodiments of the invention, a pharmaceutically active ingredient for treatment of a bladder disorder is incorporated into the biocompatible mucoadhesive thermoreversible hydrogel, wherein said disorder is characterized by bladder spasms. In some embodiments of the invention, said disorder is selected from the group consisting of urinary incontinence due to unstable bladder or unstable detrusor sphincter; voiding complications due to detrusor overactivity or a hypertrophied bladder neck; neurogenic bladder dysfunction secondary to conditions such as Parkinson's disease, spinal cord injury, stroke or multiple sclerosis; and bladder pathologies characterized by a spasm reflex, overactive bladder, interstitial cystitis, stress incontinence, urge incontinence, or neurogenic bladder.

The pharmaceutically active agent for treatment of a bladder disorder incorporated into said biocompatible mucoadhesive thermoreversible hydrogel may be incorporated into the biocompatible mucoadhesive thermoreversible hydrogel immediately prior to administration of said pharmaceutically active agent.

Further disclosed herein is the use, in a composition for treatment of neurological pathologies of the skeletal, cardiac, or smooth muscles, of a biocompatible mucoadhesive thermoreversible hydrogel composition into which a pharmaceutically active agent for treatment of a neurological pathology of the skeletal, cardiac, or smooth muscles, has been incorporated. As disclosed herein said treatment comprises applying said composition to said muscle and/or tissue surrounding said muscle by means other than intramuscular injection. As disclosed herein, said pathology is Irritable Bowel Syndrome.

As such, the compositions disclosed herein can be used in treating a neurological pathology of the skeletal, cardiac, or smooth muscles, by applying to said muscles and/or the tissue surrounding them a biocompatible mucoadhesive thermoreversible hydrogel into which a pharmaceutically active agent for treating said neurological pathology has been incorporated, wherein said application does not comprise any step of injecting intramuscularly said pharmaceutically active agent, As disclosed herein, said pathology is Irritable Bowel Syndrome.

In some embodiments the biocompatible mucoadhesive thermoreversible hydrogel comprises at least one reverse thermal gelation agent and at least one component selected from the group consisting of mucoadhesive enhancers and thickening compounds.

The reverse thermal gelation agent is a Poloxamer. In some embodiments of the invention, said reverse thermal gelation agent comprises a Poloxamer selected from the group consisting of Poloxamer 407, Poloxamer 188, and Poloxamer 338. The reverse thermal gelation agent may comprise a pH-controlled gelation agent selected from the group consisting of cellulose, acetophthalate, and carbomers.

In the embodiments of the invention, said component selected from the group consisting of mucoadhesive enhancers and thickening compounds is selected from the group consisting of polycarbophil; polyacrylic acids; crosslinked acrylic acid; polymethacrylates; divinyl glycol; polyethylene glycol (PEG); polyethylene oxide; vinylpyrrolidone/vinyl acetate copolymer; cellulose; microcrystalline cellulose; cellulose derivatives; gelatin; starch; starch derivatives; gums; dicalcium phosphate; lactose; sucrose; polyvinylpyrrolidone (PVP); polyvinyl alcohols (PVA); partially hydrolysed polyvinyl acetate (PVAc); polysaccharides; waxes; fats; fatty acid derivatives; hyaluronic acid; chitosan; κ-carrageenan; carbomers; Eudragit; and any combination thereof.

Non-limiting examples of cellulose derivatives that are suitable for use in the mucoadhesive enhancer / thickening compound component include methylcellulose, ethylcellulose, HPMC, methoxy-substituted HPMC with any possible degree of methoxy substitution (from 0 to 3), hydroxy-propylcellulose (HPC), other hydroxyalkylcelluloses, hydroxyalkylmethylcelluloses, low-substituted hydroxypropylcellulose (L-HPC), hydroxypropymethylcellulose acetate succinate (HPMCAS), carboxymethylcelluloses and salts thereof. Non-limiting examples of starch derivatives that are suitable for use in the mucoadhesive enhancer / thickening compound component include mono- and diphosphate ester starches. Non-limiting examples of gums that are suitable for use in the mucoadhesive enhancer / thickening compound component include guar gum, locust beam gum, xanthan gum, and any combination thereof. Non-limiting examples of polysaccharides that are suitable for use in the mucoadhesive enhancer / thickening compound component include alginic acid, alginates, alginate esters, and galactomannans.

In some embodiments of the invention, said biocompatible mucoadhesive thermoreversible hydrogel comprises at least one component selected from the group consisting of mucosal tissue permeability enhancers; fatty acids; nonionic surfactants; essential oils, terpenes, and terpenoids; oxazolidinones (in some embodiments of the invention, said oxazolidinone is 4-decyloxazolidin-2-one); cyclodextrin; pH modifiers/stabilizers; plasticizers; and swellable excipients. In some embodiments of the invention, the nonionic surfactant is selected from the group consisting of polyoxyethylene-2-oleyl ether and polyoxyethylene-2-stearyl ether. In some embodiments of the invention, said fatty acid is selected from the group consisting of lauric acid, myristic acid, oleic acid, and capric acid. In some embodiments of the invention, said essential oils, terpenes, and terpenoids are selected from the group consisting of eucalyptus oil, chenopodium oil, ylang-ylang oil and menthol.

Non-limiting examples of suitable mucosal tissue permeability enhancers include dimethylsulfoxide (DMSO); laurocapran; dimethylformamide (DMF); dimethylacetamide (DMAC); urea; n-methyl-2-pyrrolidone; other pyrrolidones; arginine; polyarginine; ethanol; nonionic surfactants (in some embodiments of the invention, the nonionic surfactant is a polysorbate); anionic surfactants; and glycols. Non-limiting examples of suitable glycols include diethylene glycol and tetraethyleneglycol.

In some embodiments of the invention, said pH modifier/stabilizer is selected from the group consisting of acids, bases and buffers. Non-limiting examples of suitable pH modifiers/stabilizers include adipic acid, malic acid, L-arginine, ascorbic acid, aspartic acid, benzenesulphonic acid, benzoic acid, succinic acid, citric acid, ethanesulphonic acid, 2-hydroxyethanesulphonic acid, fumaric acid, gluconic acid, glucuronic acid, glutamic acid, maleic acid, malonic acid, methanesulphonic acid, toluenesulphonic acid, trometamol, tartaric acid, tartaric acid, and salts thereof, preferably sodium or potassium salts.

In some embodiments of the invention, said plasticizer is selected from the group consisting of citric acid derivatives (in some embodiments, the citric acid derivative is selected from the group consisting of triethyl citrate, tributyl citrate, and acetyl triethyl citrate), phthalic acid derivatives (in some embodiments, the phthalic acid derivative is selected from the group consisting of dimethyl phthalate, diethyl phthalate, and dibutyl phthalate), benzoic acid and benzoic esters, other aromatic carboxylic esters, trimellithic esters, aliphatic dicarboxylic esters, dialkyladipates, sebacic esters (in some embodiments, diethyl sebacate), tartaric esters, glycerol monoacetate, glycerol diacetate, glycerol triacetate, polyols, glycerol, 1,2-propanediol, polyethylene glycol of a predetermined chain length, fatty acids and derivatives thereof, glycerol monostearates, acetylated fatty acid glycerides, castor oil and other natural oils and their derivatives, miglyol, fatty acid alcohols, cetyl alcohol, cetylstearyl alcohol and any combination thereof.

In some embodiments of the invention, said swellable excipient is selected from the group consisting of polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC), cellulose acetate, ethylcellulose, polymethacrylates, high-molecular weight polyethylene oxides, xanthan gum, copolymers of vinylpyrrolidone and vinyl acetate, polyvinylpyrrolidones, crospovidones, crosslinked sodium carboxymethylcellulose, poly(hydroxyalkyl methacrylate), alginates and galactomannans and mixtures thereof.

In some embodiments of the invention, said biocompatible mucoadhesive thermoreversible hydrogel comprises Poloxamer ; at least one substance selected from the group consisting of carboxymethylcellulose (CMC) and hydroxypropylmethylcellulose (HPMC); and the balance water.

In some embodiments of the invention, said biocompatible mucoadhesive thermoreversible hydrogel comprises Poloxamer ; at least one substance selected from the group consisting of CMC and HPMC; polyethylene glycol; and the balance water.

In some embodiments of the invention, said biocompatible mucoadhesive thermoreversible hydrogel comprises between 20% and 30% Poloxamer between 0.05% and 0.5% HPMC; between 0.1% and 2.5% PEG; and the balance water.

In some embodiments of the invention, said biocompatible mucoadhesive thermoreversible hydrogel comprises between 20% and 30% Poloxamer; between 0.1% and 0.3% HPMC; between 0.1% and 1.8% PEG; and the balance water.

In some embodiments of the invention, said biocompatible mucoadhesive thermoreversible hydrogel comprises 27% Poloxamer 407; 0.2% HPMC; 1% PEG; and the balance water.

In some embodiments of the invention, said biocompatible mucoadhesive thermoreversible hydrogel comprises 20% Poloxamer 407; 0.2% sodium carboxymethylcellulose; 0.5% polyethylene glycol MW 20,000; citric acid - citrate buffer (50 mmol L⁻¹, pH 5.2); and the balance water.

In some embodiments of the invention, said biocompatible mucoadhesive thermoreversible hydrogel comprises between 20% and 30% Poloxamer 407; between 0.05% and 0.8% CMC; between 0.1% and 2.5% PEG; and the balance water.

In some embodiments of the invention, said biocompatible mucoadhesive thermoreversible hydrogel comprises between 20% and 30% Poloxamer 407; between 10% and 20% Poloxamer 188; between 0.05% and 2% CMC; between 0.1% and 2.5% PEG; and the balance water.

In some embodiments of the invention, said biocompatible mucoadhesive thermoreversible hydrogel comprises 20% Poloxamer 407; 16% Poloxamer 188; 0.2% sodium carboxymethylcellulose; 1% polyethylene glycol MW 3000; and the balance water.

In some embodiments of the invention, said biocompatible mucoadhesive thermoreversible hydrogel comprises between 20% and 30% Poloxamer 407; between 10% and 20% Poloxamer 188; between 0.05% and 0.8% κ-carrageenan; between 0.1% and 2.5% PEG; and the balance water.

In some embodiments of the invention, said biocompatible mucoadhesive thermoreversible hydrogel comprises 20% Poloxamer 407; 10% Poloxamer 188; 0.3% κ-carrageenan; 1% PEG-400;and the balance water.

In some embodiments of the invention in which said biocompatible mucoadhesive thermoreversible hydrogel comprises PEG, said PEG comprises a PEG chosen from the group consisting of PEG-400, PEG-3000, and PEG-20000.

In the embodiments of the invention, the pharmaceutically active ingredient incorporated into the biocompatible mucoadhesive thermoreversible is botulinum toxin. In some embodiments of the invention, said pharmaceutically active agent is selected from the group consisting of botulinum toxin A, botulinum toxin B, botulinum toxin C₁, botulinum toxin D, botulinum toxin E, botulinum toxin F and botulinum toxin G. In some embodiments of the invention, said biocompatible mucoadhesive thermoreversible hydrogel comprises 0.2 - 20 U/kg body weight of botulinum toxin.

In some embodiments of the invention, said biocompatible mucoadhesive thermoreversible hydrogel is characterized by a viscosity of less than 200 Pa·s at at at least one predetermined temperature between 10 °C and 33 °C and a viscosity greater than 3000 Pa·s at 37 °C.

In some embodiments of the invention, said biocompatible mucoadhesive thermoreversible hydrogel is characterized by an instillation temperature of between 20 °C and 42 °C.

In some embodiments of the invention, said biocompatible mucoadhesive thermoreversible hydrogel is characterized by an instillation temperature of between 4 °C and 60 °C.

In some embodiments of the invention, said biocompatible mucoadhesive thermoreversible hydrogel is characterized by a gel point below 33 °C.

It is within the present disclosure that any step that recites a hydrogel (e.g., "obtaining a biocompatable mucoadhesive thermoreversible hydrogel," "incorporating into a biocompatible mucoadhesive thermoreversible hyrogel," etc.), is considered to be reciting a hydrogel as defined in any of the above.

### BRIEF DESCRIPTION OF THE DRAWINGS

The invention disclosed herein will now be described with reference to the drawings, wherein:
FIG. **1** presents a schematic illustration of the muscular paresis grading scale;
FIG. **2** presents graphs showing the amplitude and inter-contraction interval of bladder contractions in a control group of rats following treatment with saline or one of four thermoreversible hydrogel formulations; and,
FIG. **3** presents graphs showing the contraction amplitude and inter-contraction intervals of bladder hyperactivity (chronic bladder cystitis) induced by intraperitoneal injection of cyclophosphamide in rats, showing suppression of bladder hyperactivity by treatment with botulinum toxin, and the improved suppression of bladder hyperactivity when the botulinum toxin is administered within a thermoreversible hydrogel according to several non-limiting embodiments of the invention disclosed herein.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figures and described in the specification, but only as indicated in the accompanying claims, with the proper scope determined only by the broadest interpretation of said claims.

International (PCT) patent applications PCT/IL2011/000069 (henceforth **'069**) and PCT/IL2012/000284 (henceforth **'284)**, disclose a family of biocompatible thermoreversible hydrogel compositions for sustained-release application of therapeutic agents. The hydrogel compositions disclosed comprise, in some embodiments, a Poloxamer (a triblock copolymer made up of a chain of polyoxypropylene flanked by two chains of polyoxyethylene), hydroxypropylmethylcellulose (HPMC), and polyethylene glycol (PEG). The HPMC concentration is much lower (generally less than 0.5% w/w) than is found in typical hydrophilic thermoreversible hydrogels. The compositions disclosed in **'069** and **'284** have sufficiently low viscosities at low temperatures that they can be administered into a body cavity but are gels at body temperature. Furthermore, they have a sufficiently high mucoadhesiveness that they remain attached to the interior of the body cavity long enough (weeks in some embodiments) to be able to provide sustained-release delivery of a therapeutic agent incorporated therein.

Unless otherwise stated, all concentrations are given as percentage w/w.

As used herein, the term "Poloxamer" refers to an ethylene oxide - propylene oxide triblock copolymer.

As used herein, the term "HPMC" refers to hydroxypropylmethylcellulose.

As used herein, the term "CMC" refers to carboxylmethylcellulose.

As used herein, the term "PEG" refers to polyethylene glycol.

The inventors of the present compositions have discovered, surprisingly, that these hydrogels provide a means for maintaining and/or enhancing the potency and/or efficacy of Botulinum toxin (A, B, C₁, D, E, F and G) and equivalent neurotoxins or other therapeutic agents for treatment of bladder disorders (non-limiting examples include anticholinergic and antimuscarinic drugs such as oxybutynin, tolterodine, fesoterodine fumarate, solifenacin, and beta-3 agonists such as mirabegron) during sustained-release delivery. Relative to the methods known in the art for introduction of such therapeutic agents into the bladder, the hydrogel compositions increase the treatment efficacy of, and enhance the therapeutic effect of, these therapeutic agents. The additional efficacy is particularly useful in treatment of disorders characterized by bladder spasms, non-limiting examples of which include urinary incontinence due to unstable bladder or unstable detrusor sphincter; voiding complications due to detrusor overactivity or a hypertrophied bladder neck; neurogenic bladder dysfunction secondary to conditions such as Parkinson's disease, spinal cord injury, stroke or multiple sclerosis; and bladder pathologies characterized by a spasm reflex, overactive bladder, interstitial cystitis, stress incontinence, urge incontinence, or neurogenic bladder.

In a preferred embodiment, the hydrogel into which one or more therapeutic agents is incorporated is used in a system in which it is applied to the bladder cavity without involving injection to the bladder wall. The system comprises a mucoadhesive, bioerodible, biocompatible hydrogel with reverse thermal gelation properties that can be supplied with the active pharmaceutical agent already incorporated, or with the active pharmaceutical agent supplied separately and incorporated into the hydrogel immediately before treatment (e.g. at the patient's bedside). The system loaded with the pharmaceutical agent is inserted into the bladder cavity by a catheter without involving injection to the bladder wall. In the bladder, the system solidifies, stabilizes the pharmaceutical agent and forms a reservoir inside the bladder. The pharmaceutical agent is delivered to the bladder walls via direct contact with the mucoadhesive reservoir or by dissolution or erosion of the system providing a uniform total tissue contact/coverage (as opposed to delivery by local injection). The resulting topical concentration of the pharmaceutical agent can be controlled by altering the concentration of the pharmaceutical agent within the system or by controlling the release kinetics. Non-limiting examples of how the release kinetics can be controlled include control of the amount of pharmaceutical agent released per unit time and control of exposure time by appropriately setting the concentrations of the components of the gel in order to achieve specific desired properties such as dissolution rate and mucoadhesiveness.

Because the invention herein disclosed is more conveniently and comfortably used than compositions and methods known in the prior art, it provides improved patient compliance and comfort. The resulting topical drug concentration can be controlled by altering the hydrogel composition thus controlling the drug release kinetics, both control of drug quantity released per unit time and control of the total exposure time. The hydrogel composition is eroded and excreted from the bladder by natural urination processes.

In preferred embodiments of the invention, the biocompatible thermoreversible hydrogel comprises at least one thermal gelation agent which is a Poloxamer, at least one mucoadhesive agent, and water. In more preferred embodiments of the invention, the thermal gelation agent is a poloxamer (ethylene oxide - propylene oxide triblock copolymer) selected from the group consisting of Poloxamer 407, Poloxamer 188, and Poloxamer 338, and the mucoadhesive polymer comprises at least one component selected from the group consisting of carboxymethylcellulose (CMC), hydroxypropylmethylcellulose (HPMC), and κ-carrageenan. In yet more preferred embodiments of the invention, the hydrogel comprises between 0.05% and 0.5% CMC/HPMC component; in the most preferred, it comprises between 0.1% and 0.3% CMC/HPMC component. In some embodiments of the invention, the hydrogel additionally comprises polyethylene glycol (PEG). In preferred embodiments in which the hydrogel comprises PEG, it comprises no more than 2.5% PEG. In more preferred embodiments in which the hydrogel comprises PEG, it comprises no more than 1.8% PEG.

In some preferred embodiments of the invention, a therapeutically effective amount of botulinum toxin is incorporated into a thermoreversible hydrogel composition comprising 20 - 30% poloxamer; 0.1 - 0.3% HPMC; 0.1% - 1.8% PEG; and the balance water. In other preferred embodiments of the invention, a therapeutically effective amount of botulinum toxin is incorporated into a thermoreversible hydrogel composition comprising 20 - 30% poloxamer; 0.05 - 0.5% HPMC; 0.1 - 2.5% PEG; and the balance water. In other preferred embodiments of the invention, said therapeutically effective amount of botulinum toxin is incorporated into a thermoreversible hydrogel composition comprising 27% Poloxamer 407; 0.2% HPMC; 1% PEG; and the balance water. In other preferred embodiments of the invention, said therapeutically effective amount of botulinum toxin is incorporated into a thermoreversible hydrogel composition comprising 20% Poloxamer 407; 0.2% sodium carboxymethylcellulose; 0.5% polyethylene glycol MW 20,000; citric acid - citrate buffer (50 mmol L⁻¹, pH 5.2); and the balance water.

In other preferred embodiments of the invention, said therapeutically effective amount of botulinum toxin is incorporated into a thermoreversible hydrogel composition comprising between 20% and 30% Poloxamer 407; between 0.05% and 0.8% CMC; between 0.1% and 2.5% PEG; and the balance water. In other preferred embodiments of the invention, said therapeutically effective amount of botulinum toxin is incorporated into a thermoreversible hydrogel composition comprising between 20% and 30% Poloxamer 407; between 10% and 20% Poloxamer 188; between 0.05% and 2% CMC; between 0.1% and 2.5% PEG; and the balance water.

In other preferred embodiments of the invention, a therapeutically effective amount of botulinum toxin is incorporated into a thermoreversible hydrogel composition comprising 20% Poloxamer 407; 16% Poloxamer 188; 0.2% sodium carboxymethylcellulose; 1% polyethylene glycol MW 3000; and the balance water.

In some preferred embodiments of the invention, a therapeutically effective amount of botulinum toxin is incorporated into a thermoreversible hydrogel composition comprising between 20% and 30% Poloxamer 407; between 10% and 20% Poloxamer 188; between 0.05% and 0.8% κ-carrageenan; between 0.1% and 2.5% PEG; and the balance water. In other preferred embodiments of the invention, a therapeutically effective amount of botulinum toxin is incorporated into a thermoreversible hydrogel composition comprising 20% Poloxamer 407; 10% Poloxamer 188; 0.3% κ-carrageenan; 1% PEG-400; and the balance water.

In yet other preferred embodiments of the invention, a therapeutically effective amount of botulinum toxin is incorporated into any of the thermoreversible hydrogel compositions disclosed in **'069** or **'284**. As disclosed herein, a therapeutically effective amount of a pharmaceutically active agent other than botulinum toxin is incorporated into one of the thermoreversible hydrogel compositions disclosed above, in **'069,** or in **'284**.

In some embodiments of the invention in which said biocompatible mucoadhesive thermoreversible hydrogel comprises PEG, said PEG comprises PEG-400. In other embodiments, said PEG comprises PEG-3000. In yet other embodiments, said PEG comprises PEG-20000.

The hydrogel composition disclosed herein may additionally comprises components selected from the group consisting of aliginates, pH-controlled gelation agents such as cellulose, acetophathalate and carbomers, and any combination thereof.

In the embodiments of the invention, the hydrogel composition additionally comprises at least one component selected from mucoadhesive enhancers and thickening compounds. In the embodiments of the invention, the hydrogel composition comprises at least one component selected from adhesive and thickening compounds selected from the group consisting of polycarbophil; polyacrylic acids; crosslinked acrylic acid; polymethacrylates; divinyl glycol; polyethylene glycol (PEG); polyethylene oxide; vinylpyrrolidone/vinyl acetate copolymer; cellulose; microcrystalline cellulose; cellulose derivatives such as ethylcellulose, methoxy-substituted HPMC with any possible degree of methoxy substitution (from 0 to 3), hydroxy-propylcellulose (HPC), other hydroxyalkylcelluloses, hydroxyalkylmethylcelluloses, low-substituted hydroxypropylcellulose (L-HPC), hydroxypropymethylcellulose acetate succinate (HPMCAS), carboxymethylcelluloses and salts thereof; gelatin; starch or starch derivatives including mono- and diphosphate ester starch with any desired possible degree of substitution; gums such as guar gum, locust beam gum and xanthan gum or any combination thereof; polycarbophil; dicalcium phosphate; lactose; sucrose; polyvinylpyrrolidone (PVP); polyvinyl alcohols (PVA); partially hydrolysed polyvinyl acetate (PVAc); polysaccharides such as alginic acid, alginates, or galactomannans; waxes; fats; fatty acid derivatives; hyaluronic acid; chitosan; κ-carrageenan; carbomers; Eudragit; and any combination thereof.

In some embodiments of the invention, the hydrogel composition additionally comprises one or more of: mucosal tissue permeability enhancers such as dimethylsulfoxide (DMSO); laurocapran; dimethylformamide (DMF); dimethylacetamide (DMAC); urea; n-methyl-2-pyrrolidone or other pyrrolidones; glycols such as diethylene glycol and tetraethyleneglycol; fatty acids such as lauric acid, myristic acid, oleic acid, and capric acid; nonionic surfactants such as polyoxyethylene-2-oleyl ether and polyoxyethylene-2-stearyl ether; essential oils, terpenes and terpenoids as eucalyptus oil, chenopodium oil, ylang-ylang oil and menthol; and oxazolidinones such as 4-decyloxazolidin-2-one; cyclodextrin, arginine, polyarginine, ethanol; any combination thereof.

In some embodiments of the invention, the hydrogel composition additionally comprises at least one bonding agent. In preferred embodiments, the bonding agent is selected from the group consisting of polycarbophil, cellulose, microcrystalline cellulose, cellulose derivatives, low substituted hydroxypropylcellulose (L-HPC), dicalcium phosphate, lactose, PVP and sucrose, ethylcellulose, hydroxypropymethylcellulose acetate succinate (HPMCAS), PVP, vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides, fats and fatty acid derivatives and any combination thereof.

In some embodiments of the invention, the hydrogel additionally comprises a pH modifier/stabilizer. In preferred embodiments, the pH-modifying substance is selected from the group consisting of acids, bases and buffers. In more preferred embodiments, the pH-modifying substance is selected from the group consisting of adipic acid, malic acid, L-arginine, ascorbic acid, aspartic acid, benzenesulphonic acid, benzoic acid, succinic acid, citric acid, ethanesulphonic acid, 2-hydroxyethanesulphonic acid, fumaric acid, gluconic acid, glucuronic acid, glutamic acid, maleic acid, malonic acid, methanesulphonic acid, toluenesulphonic acid, trometamol, tartaric acid, tartaric acid, and salts thereof. In more preferred embodiments in which the pH-modifying agent comprises a salt, the salt is selected from the group comprising sodium and potassium salts.

In some embodiments of the invention, the hydrogel additionally comprises at least one matrix-forming polymer. In preferred embodiments, the at least one matrix-forming polymer is selected from the group consisting of hydroxyethylmethylcelluloses, hydroxypropylcelluloses (HPC), hydroxyethylcelluloses, methylcelluloses (MC), ethylcelluloses, alkylcelluloses, hydroxy-alkylcelluloses hydroxyalkylmethylcelluloses, sodium carboxymethylcelluloses (NaCMC), alginates, galactomannans, xanthans, polyethylene oxides, polyacrylic acids, polymethacrylic acids, polymethacrylic acid derivatives, polyvinyl alcohols (PVA), partially hydrolysed polyvinyl acetate (PVAc), polyvinylpyrrolidone (PVP), agar, pectin, gum arabic, tragacanth, gelatin, starch, starch derivatives and any combination thereof.

In some embodiments of the invention, the hydrogel additionally comprises at least one component selected from the group consisting of poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), Poloxamers of the type (poly(ethylene oxide)/poly(propylene oxide)/poly(ethylene oxide) (PEO-PPO-PEO), stearic acid, poly(acrylic acid), glyceryl stearate, cetearyl alcohol, sodium stearoyl lactylate, hydroxy-lanolin, and any combination thereof.

In some embodiments of the invention, the hydrogel additionally comprises a plasticizer. In preferred embodiments, the plasticizer is selected from the group consisting of citric acid derivatives, most prefereably triethyl citrate, tributyl citrate, or acetyl triethyl citrate; phthalic acid derivatives, most preferably dimethyl phthalate, diethyl phthalate, or dibutyl phthalate; benzoic acid and benzoic esters, other aromatic carboxylic esters, trimellithic esters, aliphatic dicarboxylic esters, dialkyladipates, sebacic esters, most preferably diethyl sebacate, tartaric esters, glycerol monoacetate, glycerol diacetate, glycerol triacetate, polyols, glycerol, 1,2-propanediol, polyethylene glycol of a predetermined chain length, fatty acids and derivatives thereof, glycerol monostearates, acetylated fatty acid glycerides, castor oil and other natural oils and their derivatives, miglyol, fatty acid alcohols, cetyl alcohol, cetylstearyl alcohol or any combination thereof.

In some embodiments of the invention, the hydrogel additionally comprises a swellable excipient. In preferred embodiments, the swellable excipient is selected from the group consisting of polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC), cellulose acetate, ethylcellulose and polymethacrylates, high-molecular weight polyethylene oxides, xanthan gum, copolymers of vinylpyrrolidone and vinyl acetate, polyvinylpyrrolidones, crospovidones, crosslinked sodium carboxymethylcellulose, poly(hydroxyalkyl methacrylate), alginates and galactomannans and mixtures thereof.

One of the critical properties of the hydrogel compositions of the present invention is their low viscosity (typically 200 Pa·s) at relatively low temperature. The specific temperature at which this low viscosity is achieved will depend on the particular conditions of use of the composition. Under typical conditions of use, a composition will be selected in which the viscosity reaches this low value at a temperature of between 10 °C and 33 °C. The compositions herein disclosed have typical viscosities of around 3000 Pa·s at body temperature, and hence will gel within the body cavity into which they are instilled.

The embodiments described in detail above, relate to the uses of hydrogels into which pharmaceutically active agents have been incorporated in compositions applicable to bladder dysfunctions. It is further within the present disclosure the use of the same hydrogels into which botulinum toxin has been incorporated in treatments for neurological pathologies of the skeletal, cardiac, or smooth muscles, such as Irritable Bowel Syndrome. In particular, these hydrogel compositions are useful in treatments in which time-released application of botulinum toxin is desired without the intramuscular injection of the botulinum toxin. As described above, the low viscosity of these compositions at low temperatures makes it possible to introduce them in the liquid form (e.g. via syringe or enema), and their high viscosity at body temperature ensures that they will gel *in situ,* enabling time-released application of the active ingredient botulinum toxin).

It is also within the present disclosure that the compositions disclosed herein are useful for treatment of neurological pathologies of the skeletal, cardiac, or smooth muscles, such as Irritable Bowel Syndrome by applying to said muscles and/or the tissue surrounding them a biocompatible mucoadhesive thermoreversible hydrogel into which a pharmaceutically active agent for treating said neurological pathology has been incorporated; (e.g. via syringe or enema), without any step of injecting intramuscularly said pharmaceutically active agent.

### EXAMPLES

The following examples are provided to illustrate various non-limiting embodiments of the invention herein disclosed. Results are presented for the use of the following four typical non-limiting hydrogel compositions (concentrations are given as percent w/w) as matrices for the delivery of a pharmaceutically active agent:

"Gel A" refers to a thermoreversible hydrogel composition comprising 27% Poloxamer 407, 0.2% hydroxypropylmethylcellulose (HPMC), 1% polyethylene glycol MW 400 (PEG-400), and water to 100%;

"Gel B" refers to a thermoreversible hydrogel composition comprising 20% Poloxamer 407, 0.2% sodium carboxymethylcellulose, 0.5% polyethylene glycol MW 20,000, citric acid - citrate buffer (50 mmol L⁻¹, pH 5.2), and water to 100%;

"Gel C" refers to a thermoreversible hydrogel composition comprising 20% Poloxamer 407, 16% Poloxamer 188, 0.2%; sodium carboxymethylcellulose, 1% polyethylene glycol MW 3000, and water to 100%; and,

"Gel D" refers to a thermoreversible hydrogel composition comprising 20% Poloxamer 407; 10% Poloxamer 188; 0.3% κ-carrageenan; 1% PEG-400; and water to 100%.

### EXAMPLE 1

Prior to testing the efficacy of botulinum toxin (BTX) incorporated into Gel A as a treatment for bladder disorders characterized by bladder spasms, the activity of BTX in the gel and following release from the gel was confirmed.

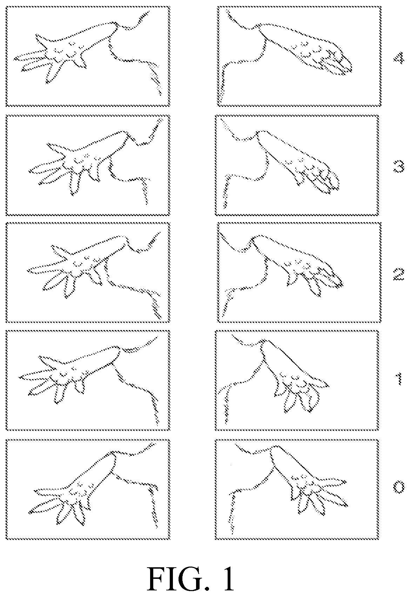

The activity of BTX was determined using single intramuscular (IM) administration of test items to mice (Hsd:ICRFemale), similar to the method disclosed in Aoki, K. R., "A Comparison of the Safety Margins of Botulinum Neurotoxin Serotypes A, B, and F in Mice", Toxicol. 2001, 39, 1815-1820. The effect of commercially available BTX-A solubilized in saline or gels, or released from gels by saline on the local muscle weakening was compared (saline injection served as negative control). Test animals were injected at a dose of about 20 U/kg body weight at a dose volume of 10 µl per animal to the right hind limb. A Digit Abduction Assay (DAS) was used to determine the local muscle weakening efficacy 48 hours after dosing. The DAS scoring was carried out by briefly suspending the animal by its tail to elicit a characteristic startle-response, namely causing the animal to extend its hind-limbs and abduct its respective hind digits. Reference is now made to FIG. 1, which depicts schematically the 5-point scale used to score the relative extent of BTX-A induced muscular paresis. According to this scale, a score of 0 represents a normal response, while a score of 4 represents the maximal reduction in digit abduction. The left column in FIG. 1 illustrates the scale for an untreated left hind leg, and the right column illustrates the scale for a treated right hind leg. The results are summarized in Table 1.

**TABLE 1**

| Test results of BTX activity following intramuscular injection | | | | |
|---|---|---|---|---|
| Group (n=10) | Right hind leg (treated) | | Left hind leg (untreated) | |
| Average score SD | Average score | SD | | |
| Saline-control | 0 | 0 | 0 | 0 |
| BTX in saline | 2.5 | 0.97 | 0 | 0 |
| BTX in Gel A | 2.3 | 1.06 | 0 | 0 |
| BTX release from Gel A | 2.3 | 1.06 | 0 | 0 |
| BTX release from Gel A | 2.3 | 1.25 | 0 | 0 |
| BTX release from Gel A, pH adjusted to 5.2 | 3 | 1.05 | 0 | 0 |

The results demonstrate that the activity of BTX injected in Gel A or released from Gel A is similar to that of BTX in saline. A slight increase in the activity was observed for BTX release from Gel A that was pH adjusted (With HCl) to 5.2 (within the test SD). Low pH is known in the literature to promote BTX stability. Hence BTX administered to the bladder in Gel A (or similar gel formulations) is expected to preserve its biological activity within the gel and following its release from the gel. Moreover the gel is expected to increase BTX stability within the bladder by preserving its three dimensional structure and adjusting its surrounding conditions (as pH) in order to ensure its maximal stability during instillation.

### EXAMPLE 2

The enhanced effect of BTX following mixing with TheraCoat systems for treatment of bladder disorders was demonstrated in a bladder cystitis rat model. The testing method is described in Chuang, Y.C.; Yoshimura, N.; Huang, C.C.; Wua, M. "Intravesical Botulinum Toxin A Administration Inhibits COX-2 and EP4 Expression and Suppresses Bladder Hyperactivity in Cyclophosphamide-Induced Cystitis in Rats," Eur. Urol. 2008, 56, 159-167. Chronic cystitis was induced by intraperitoneal injection of cyclophosphamide (75 mg/kg was injected on day 1, 4 and 7). On day two, polyethylene tubing (PE-50) was inserted into the rat bladder through the urethra. The bladder was drained and instilled with gel or saline with and without BTX (1ml, 20u/ml Allergan). On day 8 the animal was anesthetized and the bladder was examined by data requisition POWER LAB via millar transducer inserted into the bladder dome. After recovery from anesthesia the animal was gently restrained and the suprapubic catheter was connected to infusion pump (0.08 ml/min continuously) and pressure transducer for recording of intravesical pressure and for saline infusion into the bladder in order to elicit repetitive voiding. The amplitude and the inter-contraction interval of reflex bladder contractions were recorded. Each treatment group comprised 2-4 animals.

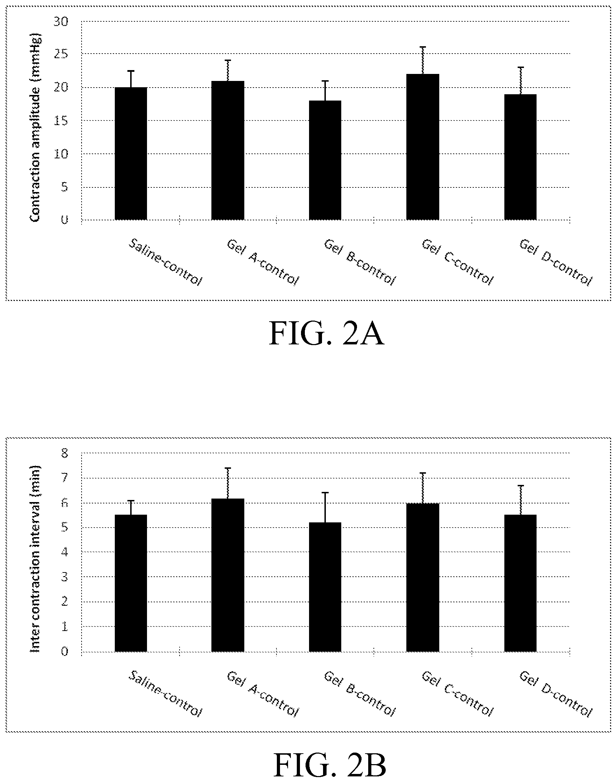

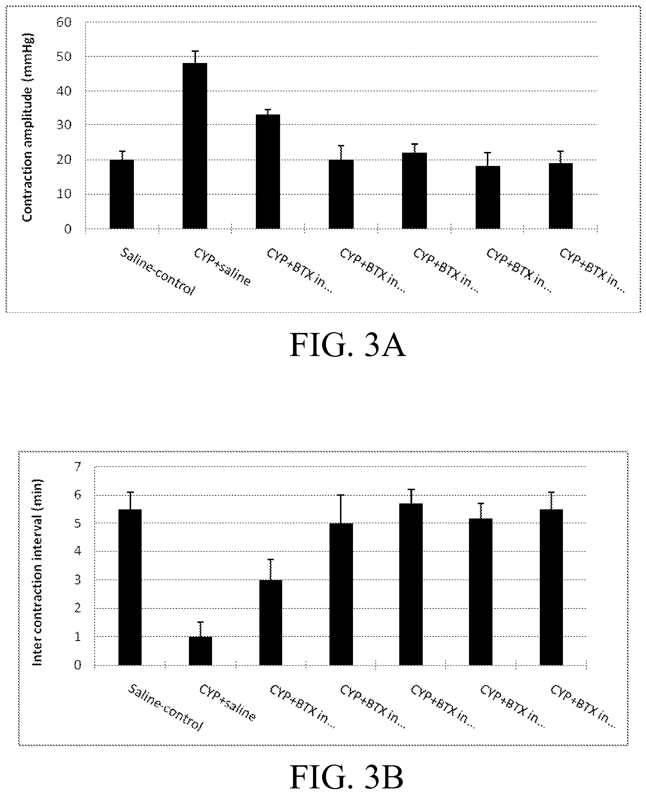

Reference is now made to FIG. **2**, which presents graphs summarizing the amplitude (FIG. **2A**) and inter-contraction interval (FIG. **2B**) of rat bladder in control experiments in which the rats were treated with saline or a gel formulation not containing any therapeutic agent. Similar results were obtained for all treatments, suggestion that the gel formulations without BTX have no effect on the bladder. Reference is now made to FIG. **3**, which presents graphs of contraction amplitude (FIG. **3A**) and inter-contraction interval (FIG. **3B**) following induction of bladder hyperactivity by intraperitoneal injection of cyclophosphamide (CYP). The bladder hyperactivity, as shown by a comparison of results for rats treated with CYP and saline to those for the control group treated by saline alone, is characterized by an increase in contraction amplitude and decrease in inter contraction interval. Rats that were administered with BTX reconstituted in saline or in gel formulations were shown to suppress the cyclophosphamide effect by decrease of contraction amplitude and increase of inter-contraction intervals. As shown in the figure, treatment by BTX incorporated into any one of the four gel formulations tested has a greater effect than treatment with BTX reconstituted in saline.

## Claims

1. A biocompatible mucoadhesive thermoreversible hydrogel composition incorporating botulinum toxin and comprising at least one reverse thermal gelation agent which is a poloxamer, and at least one component selected from the group consisting of mucoadhesive enhancers and thickening compounds, selected from the group consisting of polycarbophil, polyacrylic acids, crosslinked acrylic acid, polymethacrylates, divinyl glycol, polyethylene glycol (PEG), polyethylene oxide, vinylpyrrolidone/vinyl acetate copolymer, cellulose, microcrystalline cellulose, cellulose derivatives, gelatin, starch, starch derivatives, gums, dicalcium phosphate, lactose, sucrose, polyvinylpyrolidone (PVP), polyvinyl alcohols (PVA), partially hydrolysed polyvinyl acetate (PVAc), polysaccharides, waxes, fats, fatty acid derivatives, hyaluronic acid, chitosan, κ-carrageenan, carbomers, Eudragit, and any combination thereof for use in the treatment of a bladder disorder, wherein the bladder disorder is **characterized by** bladder spasms, wherein the treatment comprises applying the composition to a bladder cavity, and wherein the application does not comprise any step of injecting botulinum toxin into a wall of said bladder.

2. The biocompatible mucoadhesive thermoreversible hydrogel composition for the use according to claim 1, wherein said reverse thermal gelation agent comprises a poloxamer selected from the group consisting of Poloxamer 407, Poloxamer 188, and Poloxamer 338.

3. The biocompatible mucoadhesive thermoreversible hydrogel composition for use according to any preceding claim, wherein said composition comprises 27% (w/w) Poloxamer 407; 0.2% (w/w) HPMC; 1% (w/w) PEG-400; and the balance water.

4. The composition for use according to any preceding claim, wherein the botulinum toxin is selected from botulinum toxin A, botulinum toxin B, botulinum toxin C1, botulinum toxin D, botulinum toxin E, botulinum toxin F and botulinum toxin G.

5. The composition for use according to any preceding claim, wherein the disorder is selected from the group consisting of urinary incontinence due to unstable bladder or unstable detrusor sphincter; voiding complications due to detrusor overactivity or a hypertrophied bladder neck; neurogenic bladder dysfunction secondary to conditions such as Parkinson's disease, spinal cord injury, stroke or multiple sclerosis; and bladder pathologies **characterized by** a spasm reflex, overactive bladder, interstitial cystitis, stress incontinence, urge incontinence or neurogenic bladder.

## Patentansprüche

1. Biokompatible mucoadhäsive thermoreversible Hydrogelzusammensetzung, enthaltend Botulinumtoxin und umfassend mindestens ein Mittel zur inversen thermischen Gelierung, welches ein Poloxamer ist, und mindestens eine Komponente, ausgewählt aus der Gruppe bestehend aus mucoadhäsiven Verstärkern und Verdickungsverbindungen, ausgewählt aus der Gruppe bestehend aus Polycarbophil, Polyacrylsäuren, vernetzter Acrylsäure, Polymethacrylaten, Divinylglykol, Polyethylenglykol (PEG), Polyethylenoxid, Vinylpyrrolidon-/Vinylacetat-Copolymer, Cellulose, mikrokristalliner Cellulose, Cellulosederivaten, Gelatine, Stärke, Stärkederivaten, Gummis, Dicalciumphosphat, Lactose, Saccharose, Polyvinylpyrrolidon (PVP), Polyvinylalkoholen (PVA), teilweise hydrolysiertem Polyvinylacetat (PVAc), Polysacchariden, Wachsen, Fetten, Fettsäurederivaten, Hyaluronsäure, Chitosan, κ-Carrageen, Carbomeren, Eudragit und einer beliebigen Kombination davon zur Verwendung bei der Behandlung einer Blasenstörung, wobei die Blasenstörung **gekennzeichnet ist durch** Blasenkrämpfe, wobei die Behandlung das Anwenden der Zusammensetzung auf einen Blasenhohlraum umfasst, und wobei die Anwendung keinen Schritt des Einspritzens von Botulinumtoxin in eine Wand der Blase umfasst.

2. Biokompatible mucoadhäsive thermoreversible Hydrogelzusammensetzung zur Verwendung nach Anspruch 1, wobei das Mittel zur inversen thermischen Gelierung ein Poloxamer umfasst, ausgewählt aus der Gruppe bestehend aus Poloxamer 407, Poloxamer 188 und Poloxamer 338.

3. Biokompatible mucoadhäsive thermoreversible Hydrogelzusammensetzung zur Verwendung nach einem der vorstehenden Ansprüche, wobei besagte Zusammensetzung 27 Gew.-% Poloxamer 407; 0,2 Gew.-% HPMC; 1 Gew.-% PEG-400 und als Rest Wasser umfasst.

4. Zusammensetzung zur Verwendung nach einem der vorstehenden Ansprüche, wobei das Botulinumtoxin ausgewählt ist aus Botulinumtoxin A, Botulinumtoxin B, Botulinumtoxin C1, Botulinumtoxin D, Botulinumtoxin E, Botulinumtoxin F und Botulinumtoxin G.

5. Zusammensetzung zur Verwendung nach einem der vorstehenden Ansprüche, wobei die Störung ausgewählt ist aus der Gruppe bestehend aus Harninkontinenz aufgrund instabiler Blase oder instabilem Detrusor-Schließmuskel; Entleerungskomplikationen aufgrund von Detrusor-Überaktivität oder einer Hypertrophie der Harnblasenverjüngung; neurogener Blasendysfunktion sekundär zu Zuständen, wie etwa Parkinson-Erkrankung, Rückenmarksverletzung, Schlaganfall oder Multipler Sklerose; und Blasenpathologien, **gekennzeichnet durch** einen Krampfreflex, einer überaktiven Blase, interstitieller Zystitis, Stressinkontinenz, Dranginkontinenz oder neurogener Blase.

## Revendications

1. Composition d'hydrogel thermoréversible mucoadhésif biocompatible incorporant une toxine botulinique et comprenant au moins un agent de gélification thermique inverse qui est un poloxamère, et au moins un composant choisi dans le groupe constitué d'activateurs mucoadhésifs et composés épaississants, choisis dans le groupe constitué de polycarbophile, acides polyacryliques, acide acrylique réticulé, polyméthacrylates, divinyl-glycol, polyéthylène glycol (PEG), oxyde de polyéthylène, copolymère vinylpyrrolidone/acétate de vinyle, cellulose, cellulose microcristalline, dérivés de cellulose, gélatine, amidon, dérivés d'amidon, gommes, phosphate dicalcique, lactose, saccharose, polyvinylpyrrolidone (PVP), alcools polyvinyliques (PVA), acétate de polyvinyle partiellement hydrolysé (PVAc), polysaccharides, cires, matières grasses, dérivés d'acide gras, acide hyaluronique, chitosane, κ-carraghénane, carbomère, Eudragit, et n'importe quelle combinaison de ceux-ci pour utilisation dans le traitement d'un trouble de la vessie, dans laquelle le trouble de la vessie est **caractérisé par** des spasmes de la vessie, dans laquelle le traitement comprend l'application de la composition à une cavité de la vessie, et dans laquelle l'application ne comprend aucune étape d'injection de toxine botulinique dans une paroi de ladite vessie.

2. Composition d'hydrogel thermoréversible mucoadhésif biocompatible pour l'utilisation selon la revendication 1, dans laquelle ledit agent de gélification thermique inverse comprend un poloxamère choisi dans le groupe constitué de Poloxamère 407, Poloxamère 188 et Poloxamère 338.

3. Composition d'hydrogel thermoréversible mucoadhésif biocompatible pour utilisation selon l'une quelconque revendication précédente, dans laquelle ladite composition comprend 27 % (en poids) de Poloxamère 407 ; 0,2 % (en poids) de HPMC ; 1 % (en poids) de PEG-400 ; et le solde d'eau.

4. Composition pour utilisation selon l'une quelconque revendication précédente, dans laquelle la toxine botulinique est choisie parmi toxine botulinique A, toxine botulinique B, toxine botulinique C1, toxine botulinique D, toxine botulinique E, toxine botulinique F et toxine botulinique G.

5. Composition pour utilisation selon l'une quelconque revendication précédente, dans laquelle le trouble est choisi dans le groupe constitué d'incontinence urinaire à cause d'une vessie instable ou d'un détrusor-sphincter instable ; des complications mictionnelles à cause d'une suractivité du détrusor ou d'un col de vessie hypertrophié ; un dysfonctionnement neurogène de la vessie consécutif à des affections telles que la maladie de Parkinson, des lésions de la moelle épinière, des accidents vasculaires cérébraux ou la sclérose en plaques ; et des pathologies de la vessie **caractérisées par** un réflexe de spasme, une vessie hyperactive, une cystite interstitielle, une incontinence à l'effort, une incontinence par impériosité ou une vessie neurogène.
